# EUROPEAN PATENT APPLICATION

(11) **EP 2 631 007 A1**
(43) Date of publication of application: **28.08.2013**
(21) Application number: 12156459.5
(22) Date of filing: 22.02.2012
(51) Int. Cl.: B01F 11/00, G01N 33/543, G01N 35/00

(54) **Device for parallelization and performance increase in microarray-immunoassays with solid, non-porous capture-zone**

(71) Applicant: Roche Diagnostics GmbH, 68305 Mannheim (DE); F. Hoffmann-La Roche AG, 4070 Basel (CH)
(72) Inventor: Fischer, Thomas, 69231 Rauenberg (DE); Klepp, Juergen, 76676 Graben-Neudorf (DE); Lopez-Calle, Eloisa, 67059 Ludwigshafen (DE); Schierenbeck, Sigrun, 68167 Mannheim (DE); Steinmueller, Hans, 67454 Hassloch (DE)
(74) Representative: Stößel, Matthias

(57) **Abstract**

The present invention refers to a device (110) for detecting at least one analyte (112) in at least one liquid test sample (114) using a plurality of test elements (116). The device (110) comprises the plurality of test elements (116). Each of the test elements (116) individually can be brought into contact with the liquid test sample (114). Each of the plurality of test elements (116) comprises at least one analyzing zone (118) for detecting the at least one analyte (112). The device (110) further comprises a shaking unit (120). The shaking unit (120) is capable of generating a relative flow of the liquid test sample (114). The relative flow has at least one flow component perpendicular to a surface (122) of the analyzing zone (118).

## Description

### Field of the invention

The present invention refers to a device for detecting at least one analyte in at least one liquid test sample using a plurality of test elements. Further, the present invention relates to the use of the device for detecting at least one analyte in at least one liquid test sample. Moreover, the invention relates to a method for detecting at least one analyte in at least one liquid test sample.

### Related art

Immunoassays with at least one spot, e.g. as solid, non-porous capture zone for a quantitative detection of analytes are known from the prior art and have advantages like a high sensitivity and a wide measuring range at small sample volume and/or sample consumption. These immunoassays may be microarrays, e.g. protein-microarrays, which are also known as "ambient analyte" assays. They only need small amounts of capturing molecules for detecting a correct analyte concentration with the highest accuracy. By using micro spot assays up to ten times as high sensitivities can be achieved compared to a use of an ELISA (enzyme-linked immunosorbent assay) format. A microarray-assay device typically comprises one surface, e.g. at least partially made of glass and/or of plastic, on which antibodies and/or other affinity binding molecules, preferably as spot, may be immobilized. One or more spots may be attached on the surface. Usually, more spots are attached, which act as capture zone and/or which are often used for a "multiplex" detection of several analytes in a sample. For the detection of analytes, the surface is transformed with the sample comprising the analyte and immunological assay reagents. Finally, the spots, preferably at least one signal, are measured in suitable measuring setups, usually by using optical detection methods. The measured signal of the spots correlates with the amount of analyte and serves for quantification.

Lateral-flow and dot-immunoblot-formates, e.g. using porous capture zones, e.g. nitrocellulose membranes, in a comb format are disclosed e.g. in Biotechniques 1988, 6, 299-303 and in DE 3416933 A1.

Also immunoassays on non-porous capture zones in a comb-like format are known, disclosed e.g. in EP 0 408 542 A2, but as an assay similar to an ELISA assay, e.g. without spots and without a microarray.

Prior art referring to the "ambient analyte" theory is disclosed e.g. in Multi-analyte immunoassay, R. Ekins et al, J. Pharm. Biomed. Anal. 1989, 7, 155-168 and Ligand assays: from electrophoresis to miniaturized microarrays, R. Ekins et al, Clin. Chem. 1998, 44, 2015-2030.

Prior art referring to microarrays is disclosed e.g. in: Antibody Microarrays: Promises and Problems, W. Kusnezow et al, Biotechniques 2002, 33, Suppl. 14-23; Multiplexing molecular diagnostics and immunoassays using emerging microarray technologies, M.M. Ling et al, Expert Rev. Mol. Diagn. 2007, 7, 87-98; Protein Microarrays and Multiplexed Sandwich Immunoassays: What Beats the Beads?, M.F. Templin et al, Comb. Chem. & High Throughput Screening 2004, 7, 223-229; Multianalyte Microspot Immunoassay-Microanalytical "Compact Disk" of the Future, R.P. Ekins et al, Clin. Chem. 1991 37/11, 1955-1967; and Evaluating Sandwich Immunoassays in Microarray Format in Terms of the Ambient Analyte Regime, Saviranta et al, Clinical Chemistry 2004,50:10, 1907-1920.

Evaluation of Immunochromatographic Assay Systems for Rapid Detection of Hepatitis B Surface Antigen and Antibody, Dainascreen HBsAg and Dainascreen Ausab, Sato et al, Journal of Clinical Microbiology 1996, Vol. 34, No. 6, p. 1420-1422 shows an evaluation of two immunochromatographic assays (ICAs).

A Noninstrumental Immunoassay Based on Colloidal Dyes, Lubavina et al, Russian Journal of Bioorganic Chemistry 2000, Vol. 26, No.3, pp.207-212 describes the detection of pesticides by dot-immunoassay in a comb format using colloidal dyes.

Protein microarrays for diagnostic assays, Hartmann et al, Anal Bioanal Chem (2009), 393:1407-1416 reviews the current state of protein microarrays and discusses developments and future demands relating to protein arrays in their role as multiplexed immunoassays in the field of diagnostics.

Further, several test kits are commercially available. As an example, a test kit for in vitro diagnostic purposes is available under the trade name ImmunoComb^{®} II by Orgenics Ltd., Israel. The test is an indirect solid-phase enzyme immunoassay (EIA). The solid phase is a card with 12 projections ("teeth").

Prior art referring to mixing processes in the context of microarrays is disclosed e.g. in Optimal Design of Microarray Immunoassays to Compensate for Kinetic Limitations, W. Kusnezow et al, Mol. & Cellular Proteomics, 2006, 5.9, 1681-1696; Increasing robustness and sensitivity of protein microarrays through microagitation and automation, M. Hartmann et al, Anal. Chim. Acta 2006, 564, 66-73; Streptavidin-coated spot surfaces for sensitive immunoassays using fluorescence surface readout, L. Välimaa et al, Anal. Bioanal. Chem. 2008, 391, 2135-2144; Solid supports for microarray immunoassays, W. Kusnezow et al, J. Molec. Recognit. 2003, 16, 165-176.

Microarray and/or Point-of-Care immunoassays with a solid and/or non-porous capture zone, like µ-arrays and/or lateral-flow-assays, may have the advantage of high sensitivity and wide measuring range, but these assays may also show several disadvantages and/or shortcomings: A user and/or an operator may only be able to measure with and/or to work with devices known from the prior art, like lateral-flow and/or µ-array-devices, a few samples in parallel and/or simultaneously, as these devices usually may be constructed for analyzing single samples. More samples usually may be prepared and/or detected sequentially. A parallel measurement of several samples may require special skills, e.g. the possibility of simultaneous and/or optimally nested and/or optimally timed and/or optimally synchronized sample feeding and/or incubation, e.g. with assay reagents, and/or following detection. A nested measurement and/or small time slots and/or many method steps may have the disadvantages of a generation of a lot of error sources which may increase by the number of samples, e.g. as one has to take care for different time slots. A nested process may also not be able to provide a parallel measurement of a plurality of samples and one may have the disadvantage that a detection rate with state of the art lateral-flow and/or µ-array devices may be small and thus a time needed for detecting several samples may be very high. The time needed for detecting several samples may be an assay time, e.g. a turn-around-time (TAT), multiplied by the number of samples.

Protein microarray-assays usually are, as all solid phase assays, diffusion controlled, and longer incubation times may be needed, preferably for reaching an equilibrium. In general, a mass transport, e.g. a flow, may be an important influencing factor for the detection, preferably for the performance of the assay, e.g. in respect to the sensitivity and/or precision. In prior art devices, methods and uses a shaking of the sample, preferably of the liquid test sample, e.g. of a solution being in contact with the solid capturing zone, may improve the mass transport. But even the intensive mixing methods disclosed in the prior art are not optimal for achieving a high analytical sensitivity and a good precision. As a consequence, longer incubation times may be required to improve the assay performance. However, short assay times may be very important in in-vitro-diagnostic (IVD). Particularly in the point-of-care field a TAT smaller 10 minutes, preferably smaller 20 minutes may be essential. Usually, the shaking and/or mixing modes as disclosed in prior art only generate a flow of the sample, e.g. of the assay solution, parallel to a plane of the capture zone, which may also be called laminar mixing, or a non-defined flow where the laminar mixing dominates. Both may lead to moderate signal yields. A lot of mixing modes may lead to formation of patterns on the capture zones, e.g. on the capture spots, which may often be a mapping of the mixing motion und which may lead to misinterpretations, e.g. in a calculation of the signal, and therefore to a loss of precision.

### Problem to be solved

Thus, the technical problem underlying the present invention can be seen as the provision of means and methods for reliably detecting analytes in a liquid test sample with high sensitivity. Preferably, it may be an object of the present invention to provide a device and a use and a method which at least partially avoid the disadvantages and shortcomings of the devices, methods and uses known from prior art. Specifically, it is an object of the present invention to provide a method and/or a device and/or a use for effectively mixing a liquid test sample, which preferably may not only generate a laminar flow but also additionally a flow component of the liquid test sample perpendicular to a surface of an analyzing zone and/or to a surface of a capture zone. Additionally, the present invention may provide homogeneous, preferably over a complete surface of the analyzing zone and/or of the capture zone and/or of a surface of a test element and/or of a surface of a microarray, and/or reproducible mixing of the liquid test sample, e.g. of an assay solution, preferably for an achievement of homogeneous signals and thus high precision. The present invention also may provide a method for mixing which may be easy and cheap, preferably in an implementation.

### Summary of the present invention

This problem is solved by the device, the use and the method as claimed in the independent claims. Preferred embodiments of the invention which may be realized in an isolated way or in arbitrary combination, are disclosed in the dependent claims.

In a first aspect of the present invention, a device for detecting at least one analyte in at least one liquid test sample, preferably in a plurality of liquid test samples, using a plurality of test elements is disclosed. The device comprises the plurality of test elements.

As used in the present specification, the term "comprising" or grammatical variations thereof, such as the term "comprise", are to be taken to specify the presence of stated features, integers, steps or components or groups thereof, but do not preclude the presence or addition of one or more other features, integers, steps, components or groups thereof. The same applies to the term "having" or grammatical variations thereof, which is used as a synonym to the term comprising.

Preferably the analyte, most preferably a plurality of analytes, may be detected simultaneously in the liquid test sample, preferably in the plurality of liquid test samples. The term "simultaneously", as used in the present invention, may imply a time delay smaller 30 seconds, e.g. a time delay smaller 5 seconds, preferably smaller 1 second, most preferably smaller 100 ms, or even smaller 1 ms. The time delay may be a time period between the detection of the at least one analyte in at least two different liquid test samples of the plurality of liquid test samples and/or between the detection of two different analytes in the liquid test sample. Most preferably "simultaneously" may imply a parallelism in time of at least one step of the detection of at least two different analytes in the liquid test sample and/or the detection of the analyte in at least two different liquid test samples, with the time delay as range of tolerance.

Each of the test elements individually can be brought into contact with the liquid test sample, preferably with at least one liquid test sample of the plurality of liquid test samples. Each of the plurality of test elements comprises at least one analyzing zone for detecting the at least one analyte. The device further comprises a shaking unit, the shaking unit being capable of generating a relative flow of the liquid test sample, the relative flow having at least one flow component perpendicular to a surface of the analyzing zone. A time duration for detecting at least one analyte, e.g. the measuring time per analyzing zone, may be 1 ms to 20 s, e.g. depending on the analyte and/or on the amount of analyte, e.g. on the concentration of the analyte. The analyzing zones and/or the analytes may be detected simultaneously, as defined above, and/or separately, e.g. one after the other. A time for positioning the analyzing zones, e.g. microarray discs, in the device may be less than 1 s, e.g. for a manual positioning, and/or 100 ms to 5 s, e.g. for automatic positioning.

The term "individually", as used in the present invention, may imply that preferably each test element may be brought into contact with a specific liquid test sample, preferably determined by a user. Most preferably one test element may be brought into contact with one liquid test sample. One test element also may be brought into contact with more liquid test samples, either one after the other or simultaneously. Alternatively, more than one test element may be brought into contact with one liquid test sample or with more than one liquid test sample, either one after the other or simultaneously.

The term "detecting" as used herein may relate to a specific chemical and/or biological assay. The term "detecting" may comprise a qualitative and/or semiquantitative and/or quantitative determination of the analyte. The determination may provide a percentage and/or a fraction, e.g. a particle fraction, and/or a total mass and or a total volume and or a volume per volume unit and/or a mass per mass unit of the analyte, preferably in the liquid test sample. Said assay, preferably, may comprise means which allow for specifically detecting the analyte in the liquid test sample. Preferably, said means may be capable of specifically recognizing the chemical structure of the analyte and/or may be capable of specifically identifying the analyte based on its capability to react with other compounds and/or its capability to elicit a response in a biological read out system. Means which may be capable of specifically recognizing the chemical structure of an analyte may be, preferably, nucleic acids such as aptamers and/or polypeptides such as antibodies which specifically may interact with chemical structures of the analyte. Specific antibodies, for instance, may be obtained using the analyte as an antigen by methods well known in the art. Preferably, the detection may include at least one compound separation step prior to the detection step referred to before. These techniques are well known in the art and can be applied by the person skilled in the art without further ado.

In one aspect of the invention, the detection of the analyte may comprise the use of a detection antibody which specifically binds to the detection complex comprising the immobilized antibody and the analyte bound thereto. The detection antibody may be coupled to a detectable label allowing the measurement of the amount of the detection antibody which may be bound to the detection complex. By measuring the said amount of bound detection antibody, the amount of the bound analyte may be determined since the amount of bound detection antibody in the detection complex usually correlates with the amount of antibody-complex comprised by the detection complex. Labeling may be done by direct and/or indirect methods. Direct labeling may involve binding of the label directly (covalently and/or non-covalently) to the first detection antibody. Indirect labeling may involve binding (covalently and/or non-covalently) of an agent which specifically binds to the detection antibody and which carries a detectable label. Such an agent may be, e.g., a secondary (higher order) antibody which specifically binds to the detection antibody. The secondary antibody in such a case may be coupled to a detectable label. It will be understood that further higher order antibodies may be used in addition for detection of the detection complex. The higher order antibodies are often used to increase the signal. Suitable higher order antibodies may also include the well-known streptavidin-biotin system (Vector Laboratories, Inc.), and the well-known Dako LSAB™2 and LSAB™+ (labeled streptavidin-biotin), or Dako PAP (Peroxidase Anti-Peroxidase). In a further aspect, the said label of the first detection antibody may be selected from the group consisting of: fluorescent dyes, chemoluminescent molecules, radioactive labels and enzymes capable of generating a detectable signal. Typical fluorescent labels may include fluorescent proteins (such as GFP and its derivatives), Cy3, Cy5, Texas Red, Fluorescein, and the Alexa dyes (e.g. Alexa 568). Typical radioactive labels may include 35S, 125I, 32P, 33P and the like. Alternatively, a detectable label coupled to the said first detection antibody may also be an enzyme which may be capable of generating a detectable signal, e.g., by conversion of a substrate. In an aspect, such an enzyme may be a peroxidase (e.g., horseradish peroxidase) or alkaline phosphatase. In a further embodiment, the detection antibody or the secondary antibody may be labeled with a primer and by DNA amplification methods amplification products may be generated (rolling circle DNA amplification, RCA). Additionally or alternatively, particle-based labels may be used, e.g. Quantum dots and/or up-converting phosphors and/or fluorescent particles, e.g. fluorescent latex particles. The labels preferably may be or may act as markers. Particles like polystyrene beads, e.g. carrying multiple copies of optical labels, e.g. fluorescent dyes, may be applied, e.g. as signal enhancers.

The analyte, preferably, may be a physical and/or chemical substance. The term "analyte" as used herein may refer to at least one molecule and/or atom and/or ion of a specific analyte up to a plurality of molecules and/or atoms and/or ions of the said specific analyte. Preferably, it is to be understood further that a group of analytes means a plurality of chemically different molecules wherein for each analyte at least one molecule up to a plurality of molecules may be present. An analyte in accordance with the present invention may encompass all classes of organic and/or inorganic chemical compounds, preferably including those being comprised by biological material such as organisms. Preferably, the analyte in accordance with the present invention may be a small molecule compound. Preferably, in case a plurality of analytes is envisaged, it may be understood that each analyte may represent a protein and that the plurality of proteins may represent a proteome. The proteome may be the collection of proteins being comprised by an organism, an organ, a tissue or a cell at a specific time and/or under specific conditions. More preferably, in case a plurality of analytes is envisaged, it may be understood that each analyte may represent a metabolite and that the plurality of metabolites may represent a metabolome.

The metabolome may be the collection of metabolites being comprised by an organism, an organ, a tissue or a cell at a specific time and under specific conditions. The analyte may differ from the protein or metabolite which is represented thereby due to chemical modifications arising as a result of purification steps, GC derivatives or other modifications required for determination. However, the person skilled in the art will readily be able to allocate the analyte to a protein or metabolite or class of proteins or metabolites, respectively. Preferably, the analyte may be at least one antigen and/or at least one antibody. Most preferably, the analyte may be Troponin T. Preferably, a level of Troponin T in blood may be detected by using the device according to the present invention, preferably as a test of several heart disorders.

The term "liquid test sample" generally may refer to an arbitrary fluid, which might contain the at least one analyte to be detected. Preferably, the liquid test sample may comprise at least one liquid biological sample and/or at least one liquid sample comprising at least one biological component. Thus, the liquid test sample may comprise at least one body fluid, such as a body fluid selected from the group consisting of blood, serum, urine, saliva, ocular fluid, interstitial fluid. Preferably, the liquid test sample may comprise and/or be a sample of a body fluid and/or a sample of separated cells and/or a sample from a tissue and/or an organ and/or a sample of wash fluid and/or rinse fluid, e.g. obtained from an outer or inner body surface. Samples may be obtained by well known techniques and may include, preferably, scrapes and/or biopsies from the urogenital tract and/or perianal regions and/or anal canal and/or the oral cavity and/or the upper aerodigestive tract and/or the epidermis. Such samples may be obtained by use of brushes and/or (cotton) swabs and/or spatula and/or rinse fluids and/or wash fluids and/or punch biopsy devices and/or puncture of cavities with needles and/or surgical instrumentation. Preferably, the scrapes may contain mucosal cells and/or samples of blood and/or plasma and/or serum and/or urine and/or saliva and/or lacrimal fluid and/or stool. Most preferably, the sample may be a blood sample and/or a plasma sample and/or a serum sample. Tissue and/or organ samples may be obtained from any tissue and/or organ by, e.g., biopsy and/or other surgical procedures. Separated cells may be obtained from the body fluids and/or the tissues and/or organs by separating techniques such as filtration and/or centrifugation and/or cell sorting. The aforementioned samples may be, preferably, pre-treated before they may be used for the present invention. As described in more detail below, said pre-treatment may include treatments required to release and/or separate the compounds and/or to remove excessive material and/or waste. Suitable techniques may comprise centrifugation and/or extraction and/or fractioning and/or ultrafiltration and/or protein precipitation followed by filtration and/or purification and/or enrichment of compounds. Moreover, other pre-treatments may be carried out in order to provide the compounds in a form and/or concentration suitable for compound analysis. For example, if gas-chromatography coupled mass spectrometry is used in the context of the present invention, it may be required to derivatize the compounds prior to the said gas chromatography. Suitable and necessary pre-treatments may depend on the means used for carrying out the method of the invention and may be well known to the person skilled in the art. Pre-treated samples as described before may also be comprised by the term "liquid test sample" as used in accordance with the present invention.

Additionally or alternatively to a biological sample, the liquid test sample may comprise at least one type of washing buffer and/or at least one liquid with antibodies and/or antigens, most preferably, detection antibodies, and/or at least one marker, e.g. at least one fluorescent particle, preferably a fluorescent latex particle. The marker may be able to provide a change of a property marking the presence and/or the concentration of the analyte.

The term "bring into contact" as used herein may refer to bringing the aforementioned plurality of test elements comprising at least one analyzing zone and the at least one liquid test sample, preferably, the plurality of liquid test samples, in physical proximity as to allow physical and/or chemical interaction. Suitable conditions which allow for specific interaction are well known to the skilled worker. Said conditions may depend on the features of the analyzing zone and/or the solutions to be applied and may be adapted by the skilled artisan without further ado. Moreover, a time being sufficient to allow interaction may also be determined by the skilled worker without further ado.

The term "shaking unit" as used herein refers to a unit that may accelerate at least a part of the device, preferably the test element and/or the liquid test sample and/or the analyte and/or at least one other part of the device. The shaking unit may be able to generate a flow of the liquid test sample, preferably vertical to the analyzing zone. Said flow vertical to the analyzing zone may result in an increased mass transport compared to the prior art. Thus, as a consequence of the increased mass transport the sensitivity may be increased and/or the incubation time required for achieving equilibrium may be reduced. A defined and/or fixed placing of the analyzing zone may enable the incoming vertical flow, e.g. the flow of the liquid test sample, to flow around the analyzing zone after its contact with the surface, e.g. with the surface of the analyzing zone. This may also allow a homogeneous reaction of the liquid test sample, e.g. a liquid, with the analyzing zone, e.g. with at least one surface reagent and/or at least one immobilized reagent, which may improve the precision. The term "relative flow" may be related to a motion of the liquid test sample relative to the analyzing zone. The term "flow component" may be related to a velocity component and/or a momentum component of at least a part of the liquid test sample and/or of the analyte, preferably to a vectorial component. The term "flow component" may also be related to a momentum component of a sound wave, e.g. a supersonic wave. The shaking unit may be a translation stage, e.g. an automatic translation stage, preferably with at least one stepper motor, and/or a rotating device and/or a device providing supersonic waves and/or a mixer and/or a vibrating unit and/or a mixer and/or a stirrer and/or a centrifuge and/or a device applying at least one, preferably oscillating, field on the liquid test sample, e.g. at least one oscillating electric and/or magnetic field. The shaking unit may comprise at least one motor for performing at least one motion of at least a part of the shaking unit.

The term "perpendicular" may be referred to an angle between the direction of the flow component and the surface of the analyzing zone between 0° and 180°, e.g. between 45° and 135°, preferably between 80° and 100°, most preferably of 90°.

The surface of the analyzing zone may be a surface of the test element. The surface of the analyzing zone may be a plane, e.g. a plane of the test element. The surface of the analyzing zone may be flat or may be non-flat. Preferably, the surface of the analyzing zone may be a surface which may connect points at which a coupling and/or binding between the analyte and the detection antibody and/or the detection antigen and/or the detection complex may be possible, e.g. binding sites.

The term "perpendicular to the surface of the analyzing zone" may refer to a direction of the surface normal of the surface of the analyzing zone, e.g. also to a vector field of surface normals of the surface of the analyzing zone, preferably in the case the surface of the analyzing zone is non-flat. Preferably a vector field of the generated relative flow may be directed at least partially in the same directions as the vector field of the surface normals of the surface of the analyzing zone, preferably both vector fields may be as similar as possible, most preferably both vector fields may have same orientations in all points of the surface of the analyzing zone.

The term "test element" as used herein may refer to a plurality of test elements interconnected with each other. The test element may be a component of the device which may be adapted to change at least one detectable property of at least one part of the device and/or of the liquid test sample according to the presence or absence of the analyte, preferably in a qualitative detection of the analyte, and/or according to an amount of the analyte, e.g. a percentage and/or a fraction and/or a volume and/or a number of particles per volume unit of the analyte, preferably in a quantitative detection of the analyte. The change of the property preferably may be measurable and/or detectable, preferably by the device according to the present invention and/or by an additional device. The property preferably may comprise at least one physical property, more preferably at least one optical property, and most preferably at least one color and/or at least one luminescence, such as a fluorescence and/or a phosphorescence. Thus, the at least one property may be a color of at least a part of the liquid test sample and/or of at least a part of the plurality of liquid test samples and/or of the test element and/or an emission of electromagnetic waves, e.g. light, preferably by the test element and/or by the liquid test sample and/or by at least a part of the plurality of liquid test samples and/or a transparency of the liquid test sample and/or of at least a part of the plurality of liquid test samples and/or of the test element, e.g. for electromagnetic waves, preferably for light and/or a viscosity of the liquid test sample and/or of at least a part of the plurality of the liquid test samples and/or other properties. Each test element comprises at least one analyzing zone, e.g. the analyzing zone as described above. The test element may comprise at least one capture spot. Preferably, the analyzing zone may be a non-porous analyzing zone and/or the capture spot may be a non-porous capture spot. The test element may be or may comprise at least one microarray and/or at least one immunoassay and/or at least one binding assay, e.g. at least one receptor-ligand assay. The analyzing zone may be a pad of the microarray.

In a preferred embodiment, the plurality of test elements may form a comb-like spaced structure. A comb-like spaced structure may be a structure having a plurality of protrusions protruding from at least one base, such as a plurality of at least two, at least three, at least four or at least five protrusions. The protrusions, preferably, may have a uniform length. One or more test elements per protrusion may be provided. Accordingly, one or more analyzing zones per protrusion may be provided. The protrusions may also be referred to as the "teeth" of the comb-like spaced structure. Thus, the comb-like spaced structure may comprise at least two teeth. Preferably, the comb-like spaced structure may comprise a plurality of teeth. The teeth may be arranged in at least one row. The teeth may be connected to each other, preferably each tooth may have not more than two directly neighboring teeth. Alternatively each tooth may have not more than twelve directly neighboring teeth. Most preferably, the comb-like spaced structure may comprise twelve teeth. The teeth may be arranged parallel to each other. Each tooth may comprise at least one test element. The test element may be the comb-like spaced structure. The comb-like spaced structure may comprise at least one test element, preferably at least two test elements. At least one tooth, preferably two teeth of the comb-like spaced structure may comprise at least one test element, e.g. the microarray. Most preferably each tooth of the comb-like spaced structure may comprise at least one test element, e.g. the microarray. Preferably the test element, e.g. the microarray, may be arranged on a surface of the tooth and/or of the teeth, e.g. on the tip of the tooth, preferably on at least one front surface, e.g. one test element may be arranged on the front surface and/or one test element may be arranged on the back surface, most preferably one test element may only be arranged on the front surface. The microarray and/or the test element may have an averaged thickness of 1 µm to 5 mm, e.g. of 100 µm to 2 mm, preferably of 1 mm. The analyzing zone may have an averaged thickness of 1 µm to 5 mm, e.g. 500 µm to 1 mm, preferably of 800 µm. The comb-like spaced structure, preferably with the teeth, may have an averaged thickness of 1 µm to 10 mm, e.g. 100 µm to 1 mm, preferably of 500 µm. Other sizes of the microarray and/or the test element and/or the analyzing zone and/or the comb-like spaced structure may be possible.

In another preferred embodiment, each test element may comprise a plurality of analyzing zones. The term "analyzing zone" as used herein may refer to a spot-like structure and/or a pad made of any material and/or treated material suited for the immobilization of biomolecules involved in the device, e.g. in the assay, aimed at the determination of the analyte. This material may be polystyrene and/or glass and/or polypropylene and/or cyclic olefin copolymers (COC). The material preferably may be compatible with the detection method, for example for a fluorescence-based detection the material should not show autofluorescence. The color of the material may be also important for the assay performance when using optical detection methods. Possible colors are black and/or white. The material may be at least partially transparent.

The term "analyzing zone" may refer to an arbitrary part of the test element, which is adapted to change at least one detectable property when the at least one analyte is present. The at least one detectable property may comprise a physical property and/or a chemical property and/or a biological property. The analyzing zone may comprise at least one test chemistry comprising at least one reagent adapted to perform an analyte-specific detectable reaction when the at least one analyte is present. Alternatively or additionally, the analyzing zone may comprise at least one capture zone and/or binding site adapted to specifically bind to the at least one analyte to be detected. Preferably, the analyzing zone may comprise at least one reagent and/or at least one of the means and/or at least a part thereof which may be capable of specifically recognizing the chemical structure of an analyte, as listed above. Most preferably, the analyzing zone may comprise at least one antibody, preferably at least one immobilized antibody. Moreover, the analyzing zone may be capable of specifically recognizing the chemical structure of the analyte. It is to be understood that the plurality of analyzing zones comprised by each test element may be capable of specifically recognizing the chemical structure of the same analyte. Thus, by using test elements comprising a plurality of analyzing zones capable of specifically recognizing the chemical structure of the same analyte, the amount of the analyte in the liquid test sample may be determined more precisely, i.e. to a better statistically significant portion. More preferably, the plurality of analyzing zones comprised by each test element may be capable of specifically recognizing the chemical structure of different analytes. Thus, by using test elements comprising a plurality of analyzing zones capable of specifically recognizing the chemical structure of different analytes, the amount of different analytes may be determined simultaneously in the same liquid test sample.

In a preferred embodiment, the shaking unit may be designed to shake the device in a x-y-direction. The expression "x-y-direction" may refer to a x-direction and/or a y-direction. Preferably, the x-direction may be perpendicular to the y-direction. Most preferably, the x-direction and/or the y-direction may be perpendicular to the direction of the gravitational force. The shaking unit may be adapted to shake the device perpendicular, e.g. in y-direction, and/or parallel, e.g. in x-direction and/or in z-direction, to the surface of the analyzing zone. The term "parallel", as used in the present invention, may refer to an angle between +/-90°, e.g. between +/-45°, preferably between +/-10, most preferably of 0°. The shaking unit may shake in both directions simultaneously or one after the other. The shaking unit may, e.g. by shaking in x-direction and y-direction simultaneously, shake by performing a circular and/or elliptic motion and/or a motion performing a Lissajous curve, e.g. of at least a part of the shaking unit and/or of the well and/or of the liquid test sample and/or of the test element. By shaking the device in x-y-direction, a flow vertical to the analyzing zone may be generated. The shaking unit may comprise at least one xy-shaker and/or at least one x-shaker and/or at least one y-shaker and/or at least one z-shaker, wherein the letters "x", "y", "z" may indicate the possible shaking directions, in x-direction, y-direction and z-direction, respectively. The z-direction may be perpendicular to the x-direction and/or to the y-direction and/or parallel to gravity.

In a preferred embodiment, the analyzing zone may be non-porous, e.g. the analyzing zone may comprise at least one non-porous material. The analyzing zone may be non-porous or porous, e.g. as described in Microarray Technology and Applications in Environmental Microbiology, J. Zhou and D. K. Thompson, Advances in Agronomy 2004, Volume 82, 190-191. Non-porous materials may be or may be at least part of substrates, e.g. for the analyzing zone. The non-porous materials may be characterized by their low cost and/or their mechanical stability and/or their easy modification for biomolecule attachment. In general, non-porous materials, e.g. non-porous substrates, may offer a number of advantages over porous substrates. Small amounts of molecules may be deposited at precise and/or predefined positions on the substrate surface, e.g. on the surface of the analyzing zone, preferably with little diffusion, thus e.g. enabling a high-density capacity of microarrays. The binding between target and probe molecules may occur at a much faster rate on non-porous solid surfaces than on porous substrates. This may be because molecules do not have to diffuse in and out of the pores and/or a steric inhibition of the binding reaction may not be a problem. Non-porous substrates may prevent the absorption of reagents and/or samples, e.g. of the liquid test sample and/or the analyte, into pores, thus allowing unbound labeled materials to be easily removed. This may improve reproducibility, which may be good for precision, and/or may reduce background, which may be good for sensitivity. A non-porous substrate, e.g. the non-porous analyzing zone, may also have a lower intrinsic fluorescence and thus may allow the use of a highly sensitive fluorescence detection. With regard to the expression "non-porous" reference may be made to the understanding of the skilled person. Preferably, the term "non-porous" may refer to the analyzing zone being completely free of pores or at least free of pores having an average diameter or equivalent diameter of more than 10 micrometers, preferably of more than 1 micrometer and most preferably of more than 100 nanometers or even 50 nanometers. Preferably, the analyzing zone may have a porosity of less than 10%, more preferably of less than 5% or even less than 1%. Porosity may be a measure of void, e.g. empty, spaces in a material, e.g. in the material of the analyzing zone. Porosity may be a percentage of the volume of voids over the total volume. The analyzing zone may be planar or may have micro-structures, e.g. micro-pins and/or pores, on the surface, e.g. for an increase of the surface area, especially in and/or on the capture zone. The microstructure may allow to immobilize larger amounts of capture molecules, e.g. of the analyte and/or of the liquid test sample, in the capturing zone, thus e.g. achieving a higher sensitivity and/or dynamic range.

In another preferred embodiment, the analyzing zone may comprise at least one capture zone. The capture zone may be a zone comprising at least one binding site. The capture zone may at least partially be identical with the surface of the analyzing zone. At least one point at which coupling and/or binding between the analyte and the detection antibody and/or the detection antigen and/or the detection complex may be possible and/or may be comprised by the capture zone and/or by the binding site and/or by the analyzing zone.

The term "capture zone" as used herein may refer to a spot-like structure. The material and/or the materials of the capture zone may be similar to or identical with the material and/or the materials of the analyzing zone, e.g. polystyrene and/or glass and/or polypropylene and/or cyclic olefin copolymers (COC). Preferably, the test element and/or the surface of the analyzing zone and/or a disc and/or the analyzing zone may comprise at least one material. The capture zone may be produced by coating and/or binding the respective biological molecules, preferably the antibodies, on the solid test element and/or the surface of the analyzing zone and/or on a disc and/or on the analyzing zone and/or on a carrier. Preferably, the capture zone may comprise nucleic acids such as aptamers or polypeptides such as antibodies and/or receptors and/or enzymes which specifically may interact with said chemical structures of the analyte. The capture zone may alternatively or additionally comprise the antigen itself, e.g. for performing the assay in a competitive format.

The analyzing zone may comprise at least one capture zone. Thus, it is to be understood that each analyzing zone may comprise a plurality of capture zones. Moreover, the plurality of capture zones comprised by each analyzing zone may be capable of specifically recognizing the chemical structure of the same analyte or of different analytes, respectively. By using analyzing zones comprising a plurality of capture zones capable of specifically recognizing the chemical structure of the same analyte the amount of the analyte in the sample may be determined more precisely, i.e. to a better statistically significant portion. By using analyzing zones comprising a plurality of capture zones capable of specifically recognizing the chemical structure of different analytes the amount of different analytes may be determined simultaneously in the same sample.

In a preferred embodiment, the capture zone may comprise at least one immobilized antibody. The immobilized antibody may be an antibody fixed to the capture zone and/or to the analyzing zone and/or to the test element, e.g. by at least one bonding.

The term "antibody" as used herein may encompass a monoclonal antibody and/or a polyclonal antibody and/or a single chain antibody and/or a human and/or humanized and/or primatized and/or chimerized antibody and/or a bispecific antibody and/or a synthetic antibody and/or at least one fragment of any of said antibodies. Fragments of said antibodies may include Fab and/or Fv and/or scFv fragments and/or chemically modified derivatives of any of these fragments. Antibodies may be manufactured by using methods which are described, e.g., in Harlow and Lane "Antibodies, A Laboratory Manual", CSH Press, Cold Spring Harbor, 1988. Monoclonal antibodies can be prepared by the techniques originally described in Köhler 1975, Nature 256, 495, and Galfré 1981, Meth Enzymol 73, 3. Said techniques comprise the fusion of mouse myeloma cells to spleen cells derived from immunized mammals. Antibodies may be further improved by techniques well known in the art. For example, surface plasmon resonance as employed in the BIACORE(R) system may be used to increase the efficiency of phage antibodies which bind to the epitope, see Schier 1996, Human Antibodies Hybridomas 7, 97; Malmborg 1995, J. Immunol Methods 183, 7.

Said immobilization of an antibody, in principle, may be achieved, in an aspect, by reversible or non-reversible and/or direct or indirect (via linker molecules) attachment of the antibody to the capture zone. Methods for the attachment of the antibodies to the capture zone are well known in the art and comprise attachment via amino groups of lysines and/or arginines and/or attachment by way of their thiol groups and/or attachment via carbohydrate residues of the Fc region and/or attachment by biotinylated carbohydrate and/or amino groups. Further, the attachment of the antibodies to the capture zone may comprise pretreatments such as the coating of the capture zone with poly-L-lysine and/or streptavidin and/or nickel and/or the alike. In addition, the immobilization of the antibody to the capture zone may comprise secondary antibodies, which may bind to the Fc region of the antibody to be immobilized. All the mentioned immobilization techniques may also involve linkers and/or spacers in order to increase the hydrophilicity and/or the sterical accessibility of the antibody for the antigen and/or the binding capacity of the capture zone.

In another preferred embodiment, the immobilized antibody may be an antibody which specifically recognizes a cardiac Troponin, preferably Troponin T or Troponin I and, most preferably, Troponin T.

The device further may comprise at least one well for receiving the liquid test sample. The well may be a container being able to keep the liquid test sample. The well may be at least partially arranged on the shaking unit. Preferably, the well may be fixed on the shaking unit in such a way, that the shaking unit may shake the well. Preferably, the device may comprise a plurality of wells, preferably for receiving a plurality of liquid test samples. The wells may be identical or may be different, e.g. the wells may be different in type, e.g. different shapes and/or different sizes. The wells may be filled and/or may be fillable with identical liquid test samples. Preferably, the wells may be filled and/or may be fillable with different liquid test samples, e.g. with different types of liquid test samples, e.g. at least one well may be filled and/or may be fillable with liquid test samples comprising the analyte, e.g. with blood samples, and/or at least one well may be filled and/or may be fillable with the washing buffer and/or at least one well may be filled and/or may be fillable with the antibodies and/or with the antigens and/or at least one well may be filled and/or may be fillable with the detection antibodies. The wells may be filled and/or and/or may be fillable with liquid test samples with different concentration and/or with different delay times between steps, e.g. between detection steps. Preferably, one test element may be dipped and/or dippable per well.

The device and/or the well may comprise at least one vial. The vial may be a vessel, preferably at least partially made with glass and/or plastic, e.g. polypropylene and/or polystyrene and/or COC. Preferably, the vial may be a vessel having the form of a cylinder and/or capsule, e.g. opened on one side. Preferably, the plurality of liquid test samples may be placed in the vials. The wells and/or the vials may be arranged in rows and/or in columns. The wells and/or the vials may be fixed with each other and/or may be movable independently. The plurality of liquid test samples may be placed in vials.

The vials and/or the wells may be comprised in a multi-well plate. The vials and/or the wells may also be comprised in a plurality of multi-well plates. Thus, by placing the samples into vials of a multi-well plate the at least one analyte in the plurality of liquid test samples can be detected simultaneously by using the plurality of test elements. The multi-well plate may be comprised by the device. The device also may be adapted to be attached with at least one multi-well plate. The multi-well plate may be a device comprising wells and/or vials in rows and/or columns. The multi-well plate may be a plate having wells and/or vials.

Preferably, the wells and/or the vials and/or the multi-well plate may be arranged and/or designed in such way, that the comb-like spaced structure and/or the plurality of test elements and/or the test element may be dipped and/or dippable, preferably simultaneous, into the wells and/or the vials and/or the wells and/or the vials of the multi-well plate. The distances between the wells and/or the vials may be similar, preferably the same, as the distances between the teeth of the comb-like spaced structure.

The device further may comprise at least one sample holder for holding the well and/or the vial and/or the multi-well plate, for receiving the liquid test sample. Preferably, the sample holder may be a device to fix the well and/or the wells and/or the vial and/or the vials and/or the multi-well plate, preferably on the shaking unit. Preferably, the sample holder may be capable of driving the samples in x- and/or y-direction. The sample holder may hold preferably a plurality of wells, preferably for receiving a plurality of liquid test samples, which may be identical or different, e.g. in type and/or concentration. Preferably, one test element per well may be contactable with the a liquid test sample. The multi-well plate may be a preferred embodiment of the sample holder. The sample holder may comprise the multi-well plate.

The shaking unit may be adapted to shake the sample holder. The sample holder may be fixed on the shaking unit. The shaking unit may comprise at least one mount for mounting the sample holder, e.g. comprising at least one screw and/or at least one clamp. Preferably, the shaking unit may be fixed and/or attached to the sample holder at a shaking part of the shaking unit.

The shaking unit may be adapted to induce an oscillating movement of the analyzing zone relative to the liquid test sample. This may be performed by shaking the test element with the shaking unit while holding the well and/or the vial and/or the sample holder and/or the multi-well plate at rest and/or vice versa. Because of the fluidity of the liquid test sample also all components may be shaked by the shaking unit. The oscillating movement and/or the shaking of the shaking unit may have a frequency of 1 mHz to 1 MHz, preferably of 100 mHz to 10 kHz, most preferably of 1 Hz to 20 Hz. The oscillation movement may have an acceleration, e.g. an increase and/or decrease of the frequency of the oscillation movement, e.g. of 0 Hz/s to 1 MHz/s, preferably 1 Hz/s to 100 Hz/s, most preferably 10 Hz/s. Most preferably, the oscillation movement may have a frequency, e.g. an angular frequency and/or an angular velocity, of 18 Hz with an acceleration of 10 Hz/s. The acceleration may be the velocity, e.g. in a unit of the angular frequency and/or an angular velocity per time interval, with which the frequency, e.g. the angular frequency, may increase during starting oscillating and/or with which the frequency may decrease when finishing oscillating. The mentioned frequencies may be the frequencies of the oscillation movement during constant oscillation movement, e.g. the frequency may be an end frequency, preferably the frequency may be an angular velocity and/or an angular frequency, most preferably an angular end velocity. Most preferably, the shaking unit, e.g. a shaker, may work at a preferred angular end velocity of 18 Hz and with a preferred acceleration of 10 Hz/s. The oscillating movement may have a frequency spectrum also depending on the viscosity of the liquid test sample and/or the geometry of the well and/or the vial and/or the test element.

The device may comprise at least one test element holder. The test element holder may be a device able to hold one or more test elements, e.g. the comb-like spaced structure and/or may be comprised by the comb-like spaced structure. The test elements may be connected to the test element holder, e.g. by at least one clamp and/or at least one screw. The connection between the test element holder and the test element may be a reversible connection, such as by clamping and/or a permanent connection, such as by gluing. The test element connected to the test element holder preferably may be exchangeable.

In a further preferred embodiment, the analyzing zone may be spot-like. The term "spot-like" may imply a shape with a small aspect ratio, e.g. a dot, preferably with a polygonal or round shape, e.g. circularly shaped and/or elliptically shaped. The analyzing zone may also have another shape, like an elongated shape. The analyzing zone may have an average diameter in the range from about 2 mm to about 8 mm, most preferably of 4 mm. For a circularly shaped analyzing zone the diameter may be the largest direct distance between two points on the circle. The diameter for a non-circularly shaped analyzing zone may be the diameter of a circle having the same surface as the non-circularly shaped analyzing zone. The at least one capture zone may be comprised by the analyzing zone and/or may be spot-like and/or may have an average diameter in the range from 1µm to 5 mm, preferably in the range from 100 µm to 800 µm, most preferably of 700 µm.

In another embodiment, the device further may comprise at least one drive unit. The drive unit may be capable of bringing into contact, preferably reversibly, the plurality of test elements or the at least one test element with a plurality of liquid samples, wherein the plurality of liquid samples comprises the at least one liquid test sample. In addition to the at least one liquid test sample, the plurality of liquid samples may comprise one or more other types of liquids, such as one or more of solvents, rinsing fluids, buffer fluids and reagent fluids.

The drive unit may be a device for dipping the test element into the well and/or the vial and/or the multi-well plate. The drive unit may be able to perform an up and down movement, e.g. parallel to the direction of gravity, and/or a movement in x-direction, y-direction. The drive unit may drive the test elements and/or the sample holder and/or the well and/or the vial and/or the test element and/or the multi-well plate and/or the test element holder. Preferably, the drive unit may be capable of moving the plurality of test elements vertical relative to the liquid samples (in a z-direction), thus, resulting in an up or down movement of the plurality of liquid test samples. Alternatively, but nonetheless also preferred, the drive unit may be capable of moving the plurality of liquid samples horizontal relative to the liquid samples (in x- and y-direction) resulting in a movement of the plurality of liquid samples longitudinal to the plurality of liquid samples. More preferably, the drive unit may be capable of moving the plurality of liquid samples vertical and/or horizontal relative to the liquid samples. Thus, by automation the plurality of test elements may be moved down in z-direction into the wells of the multi-well plate comprising the plurality of liquid samples. After this step of contacting the plurality of test elements with the plurality of liquid samples, the plurality of test elements may be moved up in z-direction. In another step, the plurality of test elements may be moved horizontal relative to the liquid samples (in x- and y-direction). Alternatively, but nonetheless preferred, the sample holder may move the plurality of liquid samples in x- and/or y-direction. In another step the plurality of test elements may be moved down in z-direction into wells of a well plate. This well plate or multi-well plate may comprise the plurality of liquid samples, such as the at least one liquid test sample and, additionally, other types of liquid samples, such as at least one wash buffer and/or one or more reagents for the detection of the analyte and/or the like. Thus, the steps of contacting the plurality of test elements with the plurality of liquid test samples and/or washing steps and/or the detection of the analyte may be performed by automation. The drive unit may be a device able to perform movements. The drive unit may comprise at least one actuator and/or at least one linear stage, e.g. at least one translation stage and/or at least one rotation stage and/or at least one crane and/or at least one holder for mounting the test element holder and/or the test element and/or the multi-well plate and/or the sample holder and/or the well and/or the vial, e.g. the drive unit may comprise at least one bracket and/or at least one screw.

Preferably, the drive unit further may be adapted to subsequently bring the test elements or the test element into contact with different types of liquid samples, wherein the liquid samples comprise the at least one liquid test sample. The different types of liquid samples may comprise at least one liquid test sample comprising the analyte, e.g. blood and/or saliva and/or plasma and/or urine and/or another type of body fluid, and/or the wash buffer and/or at least one fluid with detection antibodies and/or antigens and/or at least one other type of fluid and/or at least one other type of liquid, e.g. water and/or solvent and/or a liquid with at least one reagent. The different types of liquid samples may be filled and/or may be fillable into the wells and/or into the vials and/or into the multi-well plate. Preferably, each row or each column of the multi-well plate may be fillable and/or may be filled with another type of liquid sample. Preferably, at least one first row of the multi-well plate may be filled and/or may be fillable with the liquid test sample comprising the analyte and/or at least one second row of the multi-well plate may be filled and/or may be fillable with the wash buffer and/or at least one third row of the multi-well plate may be filled and/or may be fillable with the fluid with detection antibodies and/or at least one fourth row may be fillable and/or may be filled with wash buffer. The number of wells per row may be the same as the number of test elements and/or as the number of teeth of the comb-like spaced structure, preferably for dipping the test elements into all wells of a row of the multi-well plate.

The drive unit may comprise at least one z-stage for dipping the test elements into the liquid sample and/or at least one xy-stage for subsequently bringing the test samples into contact with the different types of liquid samples. The z-stage may be a device for moving at least a part of the device according to the present invention in z-direction, e.g. up and/or down, and the xy-stage may be a device for moving at least a part of the device according to the present invention in x-direction and/or y-direction, e.g. left and/or right and/or back and/or forth. The xy-stage and the z-stage may be contained within one device or may be separate devices. The xy-stage may be one device or at least one x-stage combined with at least one y-stage. The z-stage may be used and/or may be useable for dipping the test element into the different types of liquid test samples and the xy-stage may be used and/or may be useable for choosing and/or changing the wells and/or the vials and/or the row and/or the columns of the multi-well plate.

In a preferred embodiment, the device may comprise at least one temperature control unit being arranged to adjust the temperature of the liquid test sample and/or another type of liquid sample. Preferably, the temperature control unit may thermally modulate the liquid test sample, e.g. a liquid test sample solution, wherein the temperature range of the liquid test sample modulated by the temperature control may be -20°C to 100°C, e.g. 0°C to 80°C, preferably 20°C to 50°C, most preferably 20°C to 37°C. The temperature control unit may be a device capable for controlling at least one temperature, e.g. a temperature of at least a part of the liquid test sample. The temperature control unit may be able to heat and/or to cool, preferably the liquid test sample, e.g. to at least one predetermined temperature. The temperature control unit may comprise at least one heater and/or at least one cooler and/or at least one air-conditioning system and/or at least one Peltier heater and/or at least one Peltier cooler. The temperature control unit may also comprise at least one closed-loop control, e.g. at least one PID control for regulating at least one temperature.

In another preferred embodiment, the device further may comprise at least one analyzing unit. The analyzing unit may be a unit being able to analyze the property of at least one part of the device and/or of the liquid test sample, preferably the property change according to the presence or absence and/or concentration of the analyte. The analyzing unit may be designed for automatically detecting the analyte(s) applied in the plurality of liquid test samples. Methods for the detection of the analyte(s) may be well known to the person skilled in the art and are also mentioned above.

In a further preferred embodiment, the analyzing unit may comprise at least one detector to carry out the detection of at least one optical signal, preferably in the analyzing zone. The detector may be a device being able to detect photons and/or the frequency and/or the frequency spectrum of electromagnetic waves and/or a being able to spatially resolve the optical signal. The detector may comprise at least one CCD chip and/or at least one CCD camera and/or at least one CMOS (Complementary Metal Oxide Semiconductor) sensor and/or at least one photodiode, e.g. an avalanche photodiode and/or at least one spectroscopic setup and/or at least one spectrometer and/or at least one optical setup and/or at least one optical device and/or at least one laser and/or at least one prism. The detector may be attached to the multi-well plate and/or to the sample holder and/or to the test element holder and/or to the test element and/or to the shaking unit and/or may be arranged separately. The optical signal may be analyzed directly in the well and/or in the multi-well plate and/or outside. The detector may be comprised by an optical setup, e.g. for performing spectroscopic measurements, e.g. saturation spectroscopy and/or absorption measurements and/or reflection measurements. The optical signal may be generated by a method for the detection of the analyte. Adequate detectors are well known to the person skilled in the art.

In a preferred embodiment, the device further may comprise at least one evaluation unit. The evaluation unit may comprise at least one data processor. The data processor and/or the evaluation unit may have implemented at least one algorithm for calculating the amount of the at least one analyte. The evaluation unit may be a component of the device according to the present invention being able to perform at least one calculation and/or at least one comparison and/or at least one calibration for detecting the analyte. The evaluation unit also may comprise at least one data logger and/or at least one memory, e.g. for data storage. The data processor may be a device for performing at least one calculation for data evaluation and/or detection of the analyte. The algorithm may be a mathematical scheme for data evaluation and/or for data storage.

Thus, if an analyzing unit for automatically detecting the analyte is applied, the data obtained by said automatically operating analyzing unit may be processed using an algorithm for calculating the amount of the at least one analyte by, e.g., a computer as evaluation unit. The computer may be comprised by the evaluation unit. Preferably, the means may be comprised by a single device in such a case or may be separate devices.

The term "amount" as used herein encompasses the absolute amount of an analyte or the relative amount and/or concentration of the said analyte and/or any value and/or parameter which may correlate thereto and/or may be derived therefrom.

The liquid test sample and/or the test element may comprise at least one reagent, preferably at least one antibody.

The reagent, e.g. an assay reagent, may be arranged in the wells and/or in the vials and/or in at least one multititer plate (MTP) and/or in the multi-well plate and/or in the sample holder. The MTP may be a sample holder, e.g. a vessel for the liquid test sample, and/or the reagent, preferably at least one assay reagent. The reagent may be added when the liquid test sample, preferably with the analyte, may be filled into the wells and/or into the vials and/or into the MTP and/or into the multi-well plate and/or into the sample holder.

Alternatively or additionally, the reagent, e.g. the assay reagent, may be stored as liquid reagent, e.g. in the multi-well plate, e.g. capped, preferably by at least one heat sealing film, which may be perforated when the liquid test sample, preferably with the analyte, may be filled into the wells and/or into the vials and/or into the MTP and/or into the multi-well plate and/or into the sample holder.

Alternatively or additionally, the reagent, e.g. the assay reagent, may be stored, e.g. as dried and/or chemical reagent, wherein the reagent may be solved, preferably by the liquid test sample itself and/or by the shaking unit and/or by at least one additional liquid.

The device may comprise at least one splash guard. The splash guard may be adapted to prevent cross-contamination of the wells and/or of the vials, preferably of the liquid test sample, e.g. during shaking and/or filling. The splash guard may comprise at least one cover.

A further aspect of the present invention, is a use of the device according to the present invention for detecting at least one analyte in at least one liquid test sample. Preferably, the device according to the present invention may be used for determining the amount of the at least one analyte in a plurality of liquid test samples for diagnosing a disease and/or a predisposition therefore. Preferably, the evaluation unit may comprise at least one database with stored reference amounts and/or at least one computer program code which when tangibly embedded on a computer may carry out a comparison of the determined amounts and the reference amounts stored in the database. More preferably, the evaluation unit may comprise at least one further computer program code which may allocate the result of the comparison, e.g. to a risk prediction. In such a case, it preferable may be that the evaluation unit may comprise at least one further database wherein the reference amounts may be allocated to the risks. The results may be given as output of raw data which may need interpretation, e.g. by a clinician. Preferably, the output of the device may, however, be processed, i.e. evaluated, raw data the interpretation of which does not require a clinician. The database referred to above may comprise a data collection on a suitable storage medium. Moreover, the database, preferably, further may comprise at least one database management system. The database management system may be, preferably, a network-based and/or hierarchical and/or object-oriented database management system. Furthermore, the database may be a federal or integrated database. More preferably, the database will be implemented as a distributed (federal) system, e.g. as a Client-Server-System. More preferably, the database may be structured as to allow a search algorithm to compare a test data set with the data sets comprised by the data collection. The data storage medium referred to above may encompass data storage media which may be based on single physical entities such as at least one CD and/or at least one CD-ROM and/or at least one hard disk and/or at least one optical storage media and/or at least one diskette. Moreover, the term further may include data storage media consisting of physically separated entities which may be operatively linked to each other in a manner as to provide the aforementioned data collection, preferably, in a suitable way for a query search. In a preferred embodiment, the liquid test sample may be at least one blood and/or serum and/or plasma sample.

In a further aspect of the present invention, a method for detecting at least one analyte in at least one liquid test sample is disclosed. The method may use the device according to the present invention.

The method may comprise the steps of:
a) providing at least one liquid test sample and a plurality of test elements;
b) contacting each of the test elements individually with the liquid test sample, wherein each of the plurality of test elements comprises at least one analyzing zone, wherein the analyzing zone detects the at least one analyte, preferably under conditions which allow for binding and/or at least one change of at least one property of at least one part of the device and/or of the liquid test sample, wherein the step comprises generating a relative flow of the liquid test sample by at least one shaking unit, the relative flow having at least one flow component perpendicular to a surface of the analyzing zone; and
c) determining binding of the said at least one analyte to the analyzing zone on each test element and/or the change of the property of at least one part of the device and/or of the liquid test sample.

The methods may comprise these steps in the given order or in another order. Each step may be performed only once or repeatedly. The method also may comprise one or more additional steps, e.g. at least one washing step and/or at least one delay step and/or at least one heating step and/or at least one cooling step.

Further, the present invention relates to a method for detecting at least one analyte in a plurality of liquid test samples, wherein a device may be used as referred to above.

Preferably the device, e.g. as assay device, and/or the method and/or the use, e.g. as assay process, may comprise at least one incubation step and/or at least one washing step and/or at least one detection and/or a at least one output, e.g. of the concentration of the analyte, wherein the steps and/or the method may be at least partially automatic, preferably completely automatic. The term "automatic" may refer to steps being processed without the help of an user and/or of an operator. Preferably, the user and/or the operator only may provide the liquid test sample.

The device and/or the use and/or the method according to the present invention may have different advantages, like an increase of a rate of detected liquid test samples and/or a suppression of sources of errors because of a simultaneous measurement and/or detection of several liquid test samples. The present invention may provide an easy and/or fast method for simultaneously and/or sensitively detecting several analytes in liquid test samples, which may at least suppress the disadvantages of prior art methods and/or uses and/or devices.

The comb-like spaced structure comprised by the device according to the present invention may lead to parallelism, thus an increase of the rate of analyte detection, preferably simultaneously with a reduction of the sources of errors.

The test element holder, e.g. a comb holder, preferably for fixing the comb-like spaced structure, e.g. a comb, in the multi-well plate, e.g. the MTP, preferably during shaking by the shaking unit and/or the direction of the mixing of the liquid test sample in the wells and/or in the vials, e.g. the generation of the relative flow of the liquid test sample having at least one flow component perpendicular to the surface of the analyzing zone, e.g. perpendicular to the surface of the test element, e.g. of the surface of a microarray, may lead to an improvement of a mass transport, preferably to the surface and/or to an increase of sensitivity, preferably at short assay times. Additionally or alternatively, the precision may be improved by the fixed and/or defined placing of the analytical zone, e.g. of the analyzing zone and/or the capture zone, via the comb holder when the liquid test sample, e.g. a liquid, may flow vertically to the surface of the analyzing zone. Thereafter the liquid test sample may flow around it.

Summarizing the ideas of the present invention, the following items are preferred:
Item 1: A device for detecting at least one analyte in at least one liquid test sample using a plurality of test elements, wherein the device comprises the plurality of test elements, wherein each of the test elements individually can be brought into contact with the liquid test sample, wherein each of the plurality of test elements comprises at least one analyzing zone for detecting the at least one analyte, wherein the device further comprises a shaking unit, the shaking unit being capable of generating a relative flow of the liquid test sample, the relative flow having at least one flow component perpendicular to a surface of the analyzing zone.
Item 2: The device according to the preceding item, wherein the device further comprises at least one well for receiving the liquid test sample.
Item 3: The device according to the preceding items, wherein the well comprises at least one vial.
Item 4: The device according to the two preceding items, wherein the vials are comprised in a multi-well plate.
Item 5: The device according to the three preceding items, wherein the device further comprises at least one sample holder for holding the well for receiving the liquid test sample.
Item 6: The device according to the two preceding items, wherein the sample holder comprises the multi-well plate.
Item 7: The device according to the two preceding items, wherein the shaking unit is adapted to shake the sample holder.
Item 8: The device according to one of the preceding items, wherein the shaking unit is adapted to induce an oscillating movement of the analyzing zone relative to the liquid test sample.
Item 9: The device according to one of the preceding items, wherein the shaking unit is adapted to shake the device perpendicular and/or parallel to the surface of the analyzing zone.
Item 10: The device according to one of the preceding items, wherein the plurality of test elements form a comb-like spaced structure.
Item 11: The device according to one of the preceding items, wherein the device comprises at least one test element holder, wherein the test elements are connected to the test element holder.
Item 12: The device according to one of the preceding items, wherein each test element comprises a plurality of analyzing zones.
Item 13: The device according to one of the preceding items, wherein the analyzing zone is non-porous.
Item 14: The device according to one of the preceding items, wherein the analyzing zone comprises at least one capture zone.
Item 15: The device according to the preceding item, wherein the capture zone comprises at least one immobilized antibody.
Item 16: The device according to the preceding item, wherein the immobilized antibody is an antibody which specifically recognizes a cardiac Troponin.
Item 17: The device according to the three preceding items, wherein the analyzing zone is spot-like and has an average diameter in the range from about 2 mm to about 8 mm and the at least one capture zone comprised by the analyzing zone is spot-like and has an average diameter in the range from about 100 µm to about 800 µm.
Item 18: The device according to one of the preceding items, wherein the device comprises at least one drive unit which is capable of bringing into contact the plurality of test elements with the plurality of liquid samples, wherein the plurality of liquid samples comprises the at least one liquid test sample.
Item 19: The device according to the preceding item, wherein the drive unit is further adapted to subsequently bring the test elements into contact with different types of liquid samples.
Item 20: The device according to the two preceding items, wherein the drive unit comprises at least one z-stage for dipping the test elements into the liquid sample and at least one xy-stage for subsequently bringing the test elements into contact with the different types of liquid test samples.
Item 21: The device according to one of the preceding items, wherein the device comprises at least one temperature control unit being arranged to adjust the temperature of the liquid test sample.
Item 22: The device according to one of the preceding items, wherein the device comprises at least one analyzing unit.
Item 23: The device according to the preceding item, wherein the analyzing unit comprises at least one detector to carry out the detection of at least one optical signal in the analyzing zone.
Item 24: The device according to one of the preceding items, wherein the device comprises at least one evaluation unit.
Item 25: The device according to the preceding item, wherein the evaluation unit comprises at least one data processor having implemented at least one algorithm for calculating the amount of the at least one analyte.
Item 26: Use of a device according to one of the preceding items for detecting at least one analyte in at least one liquid test sample.
Item 27: A method for detecting at least one analyte in at least one liquid test sample, the method using the device according to one of the preceding items referring to a device for detecting at least one analyte in at least one liquid test sample.
Item 28: The method according to the preceding item, wherein the method comprises the steps of:
   a) providing at least one liquid test sample and a plurality of test elements;
   b) contacting each of the test elements individually with the liquid test sample, wherein each of the plurality of test elements comprises at least one analyzing zone, wherein the analyzing zone detects the at least one analyte, preferably under conditions which allow for binding and/or at least one change of at least one property of at least one part of the device and/or of the liquid test sample, wherein the step comprises generating a relative flow of the liquid test sample by at least one shaking unit, the relative flow having at least one flow component perpendicular to a surface of the analyzing zone; and
   c) determining binding of the said at least one analyte to the analyzing zone on each test element and/or the change of the property of at least one part of the device and/or of the liquid test sample.
Item 29: The method according to one of the preceding method items, wherein the liquid test sample is at least one blood and/or serum and/or plasma sample.

### Short description of drawings

In the following, further potential details and features of the invention are disclosed in view of preferred embodiments, preferably in connection with the dependent claims. The features disclosed in the preferred embodiments may be realized in an isolated way or in any arbitrary combination. The invention is not restricted to the preferred embodiments. The embodiments are depicted in a figure in a schematic way. Identical reference numbers in the figures refer to identical, similar or functionally identical elements.

In the drawings:
- Figure 1: shows a perspective view of a first embodiment of a device for detecting at least one analyte in at least one liquid test sample;
- Figure 2: shows a front view of a part of a second embodiment of a device;
- Figure 3A: shows a perspective view of a third embodiment of a device and a use of the device and a method using the device; and
- Figure 3B: shows a perspective view of an embodiment of an analyzing zone of the third embodiment of the device;
- Figure 3C: shows a scheme for a use and a method of the third embodiment of the device.

### Preferred embodiments

The following examples shall merely illustrate the invention. They shall not be construed, whatsoever, to limit the scope of the invention.

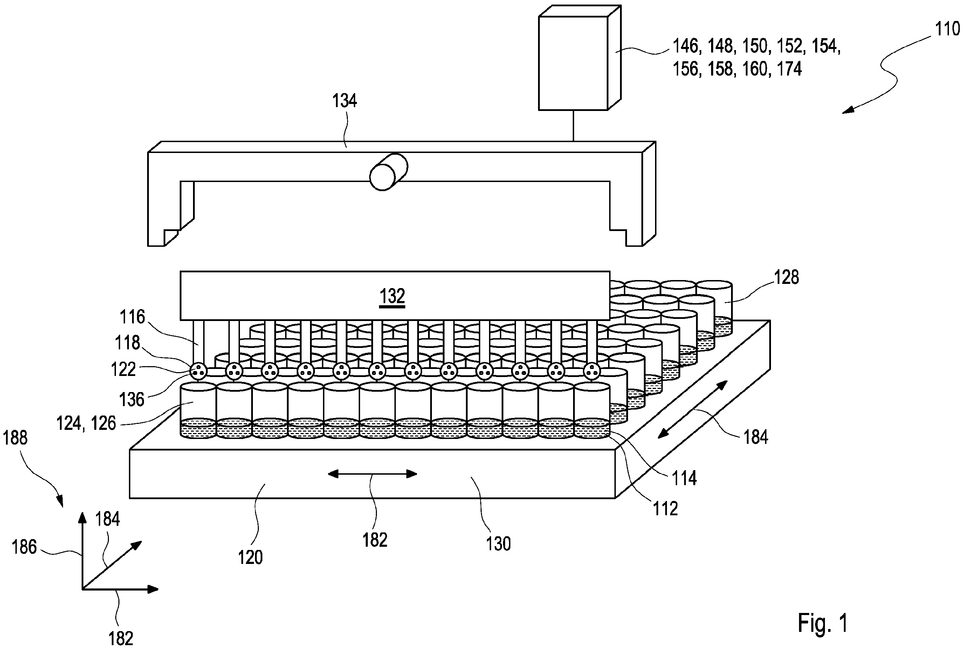

In figure 1, figure 2 and figure 3A, embodiments of a device 110 for detecting at least one analyte 112 in at least one liquid test sample 114 using a plurality of test elements 116 are shown. The device 110 may be an assay device, preferably for determining of at least one analyte 112 in at least one liquid test sample 114, e.g. in at least one liquid sample. The liquid test sample 114 may comprise e.g. blood, e.g. serum and/or plasma and/or whole blood.

The device 110 comprises the plurality of test elements 116. Each of the test elements 116 individually can be brought into contact with the liquid test sample 114. Each of the plurality of test elements 116 comprises at least one analyzing zone 118 for detecting the at least one analyte 112. The device 110 further comprises a shaking unit 120. The shaking unit 120 is capable of generating a relative flow of the liquid test sample 114. The relative flow has at least one flow component perpendicular to a surface 122 of the analyzing zone 118.

The device 110 further may comprise at least one well 124 for receiving the liquid test sample 114. The well 124 may comprise at least one vial 126. The vials 126 may be comprised in a multi-well plate 128. The device 110 further may comprise at least one sample holder 130 for holding the well 124 for receiving the liquid test sample 114. The sample holder 130 may comprise the multi-well plate 128.

The shaking unit 120 may be adapted to shake the sample holder 130. The shaking unit 120 may be adapted to induce an oscillating movement of the analyzing zone 118 relative to the liquid test sample 114. The shaking unit 120 may be adapted to shake the device 110 perpendicular, e.g. in y-direction 184, and/or parallel, e.g. in x-direction 182 and/or z-direction 186, to the surface 122 of the analyzing zone 118. The x-direction 182, y-direction 184 and the z-direction 186 are indicated in the figures by a sketched coordinate system 188.

The plurality of test elements 116 may form a comb-like spaced structure 132. Preferably, the test elements 116 may be fixed on and/or may form the at least one comb-like spaced structure 132, e.g. on a comb-like holder, preferably for detection and/or determination of more than one liquid test sample 114 and/or more than one analyte 112.

The device 110 may comprise at least one test element holder 134. The test elements 116 may be connected to the test element holder 134.

Each test element 116 may comprise a plurality of analyzing zones 118. The analyzing zone 118 may be non-porous.

The analyzing zone 118 may comprise at least one capture zone 136. The capture zone 136 may comprise at least one immobilized antibody 138. The device 110 may be based upon and/or may comprise at least one microarray immunoassay. Preferably, the device 110 and/or the capture zone 136 and/or the test element 116 may comprise immobilized antibodies 138, e.g. on a surface of at least one solid carrier. The solid carrier and/or a microarray may be embodiments of the test element 116. The capture zone 136 and/or the test element 116 may comprise at least one specific analyte binding molecule and/or at least one catching molecule, e.g. the immobilized antibodies 138, which may act as analyte binding molecules and/or as catching molecules by bringing the liquid test sample 114, e.g. a sample, into contact with the test element 116, e.g. with the solid carrier.

The immobilized antibody 138 may be an antibody which specifically may recognize a cardiac Troponin 140.

The analyzing zone 118 may be spot-like. The analyzing zone 118 may have an average diameter 142 in the range from about 2 mm to about 8 mm, preferably 4 mm. The at least one capture zone 136 may be comprised by the analyzing zone 118. The capture zone 136 may be spot-like. The capture zone 136 may have an average diameter 144 in the range from about 100 µm to about 800 µm, preferably of 700 µm. The analyzing zone 118 may have an average thickness 172 of 0.1 mm to 5 mm, preferably of 0.5 mm to 1 mm, most preferably of 0.8 mm.

The device 110 may comprise at least one drive unit 146 which may be capable of bringing into contact the plurality of test elements 116 with the plurality of liquid test samples 114. The drive unit 146 may further be adapted to subsequently bring the test elements 116 into contact with different types of liquid test samples 114. The drive unit 146 may comprise at least one z-stage 148 for dipping the test elements 116 into the liquid test sample 114. The drive unit 146 may comprise at least one xy-stage 150 for subsequently bringing the test elements 116 into contact with the different types of liquid test samples 114.

The device 110 may comprise at least one temperature control unit 152. The temperature control unit 152 may be arranged to adjust the temperature of the liquid test sample 114. The device 110 may comprise at least one analyzing unit 154. The analyzing unit 154 may comprise at least one detector 156 to carry out the detection of at least one optical signal, preferably in the analyzing zone 118. The device 110 may comprise at least one evaluation unit 158. The evaluation unit 158 may comprise at least one data processor 160. The data processor 160 may have implemented at least one algorithm for calculating the amount of the at least one analyte 112. The device 110 may be a comb assay. The device 110 may be a device 110 for simultaneously analyzing and/or processing a plurality of liquid test samples 114, e.g. up to twelve samples.

Figure 1 shows preferred components of the device 110. The first embodiment of the present invention sketched in figure 1 comprises the at least one test element holder 134, preferably one comb holder, and/or at least one comb, e.g. as the comb-like spaced structure 132, preferably comprising twelve test elements 116, e.g. twelve microarrays, and/or the at least one sample holder 130, preferably the at least one MTP, preferably with and/or fillable with the liquid test samples 114 with at least one analyte 112, e.g. a sample, and optionally at least one reagent 170, preferably in wells 124, and/or the at least one shaking unit 120, e.g. at least one shaker. The device 110 also may comprise different additional components, preferably the drive unit 146 and/or the z-stage 148 and/or the xy-stage 150 and/or the temperature control unit 152 and/or the analyzing unit 154 and/or the detector 156 and/or the evaluation unit 158 and/or the data processor 160, which are also sketched schematically in figure 1, and/or at least one other component not mentioned here.

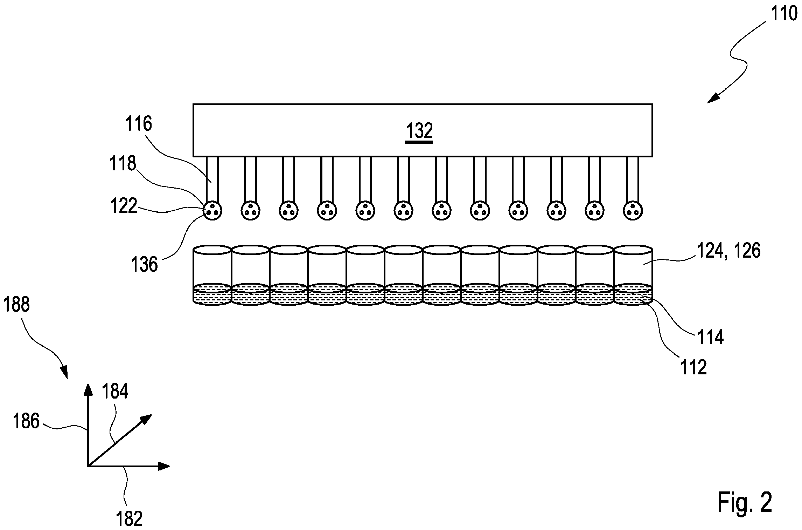

Figure 2 shows a second embodiment of the device 110 according to the present invention. The device 110 may provide parallel processing of twelve liquid test samples 114, preferably in a heterogeneous immunoassay, e.g. by sequential dipping of the comb-like spaced structure 132, e.g. a comb, in a multi-well plate 128, e.g. a 96-well-MTP. The device 110 may combine at least one test element holder 134, e.g. at least one plastic holder, preferably comprising plastic, preferably with twelve teeth. The test element 116 may comprise twelve µ-array substrates, preferably comprising at least one capture spot. The device 110 may comprise and/or may be capable for twelve samples, e.g. liquid test samples 114, and/or twelve assay reagents, e.g. the reagents 170.

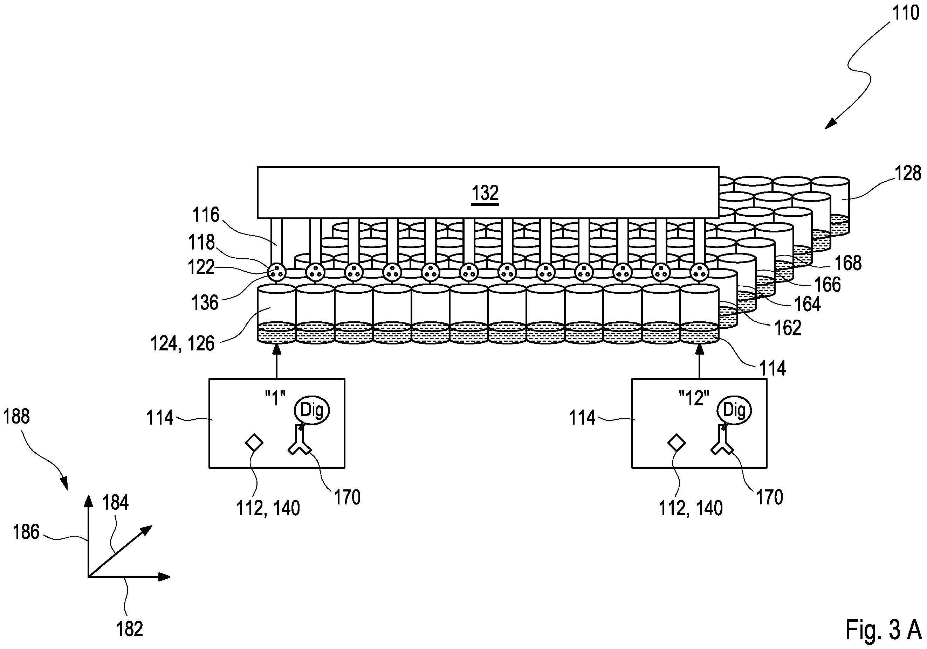

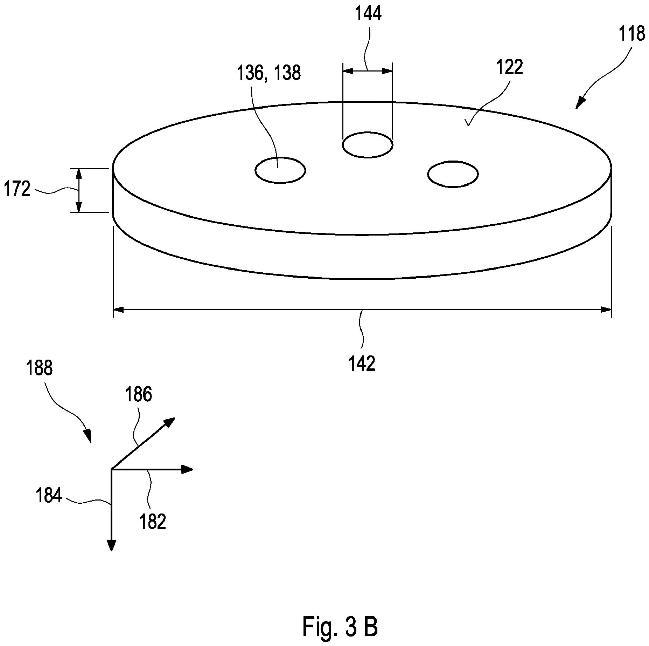

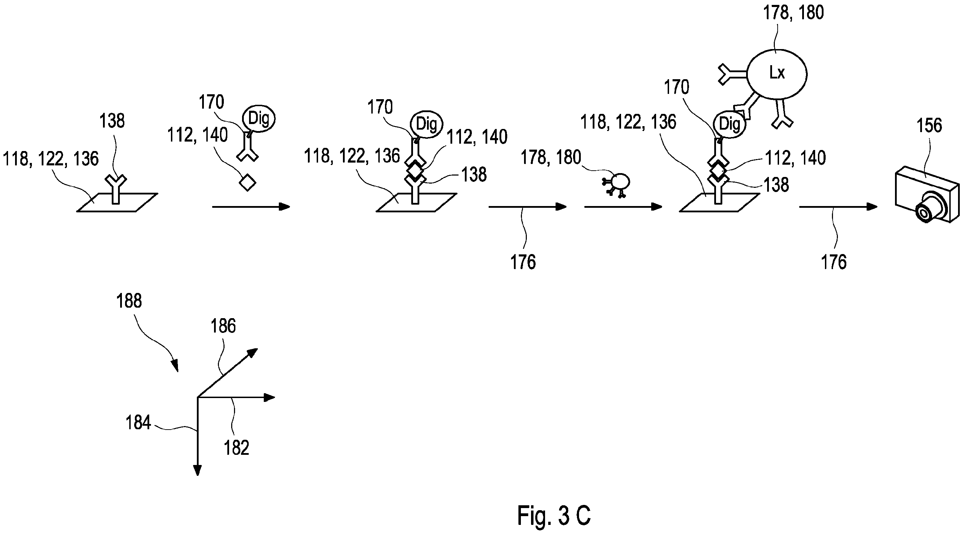

In figures 3A, 3B and 3C, an embodiment of a device, a use of the device 110 for detecting the at least one analyte 112 in the at least one liquid test sample 114 and a method for detecting the at least one analyte 112 in the at least one liquid test sample 114 is sketched. The method may use the device 110 according to the present invention for detecting the at least one analyte 112 in the at least one liquid test sample 114.

The method may comprise the steps of:
a) providing at least one liquid test sample 114 and a plurality of test elements 116;
b) contacting each of the test elements 116 individually with the liquid test sample 114, wherein each of the plurality of test elements 116 may comprise at least one analyzing zone 118, wherein the analyzing zone 118 may detect the at least one analyte 112, preferably under conditions which may allow for binding and/or at least one change of at least one property of at least one part of the device 110 and/or of the liquid test sample 114, wherein the step may comprise generating a relative flow of the liquid test sample 114 by at least one shaking unit 120, the relative flow may have at least one flow component perpendicular to a surface 122 of the analyzing zone 118; and
c) determining binding of the said at least one analyte 112 to the analyzing zone 118, preferably on each test element 116, and/or the change of the property of at least one part of the device 110 and/or of the liquid test sample 114.

The methods may comprise these steps in this order or in another order. Each step may be performed only once or more times. The method also may comprise at least one more step, e.g. at least one washing step 176 and/or at least one delay step and/or at least one heating step and/or at least one cooling step.

For bringing the liquid test samples 114 into contact with the test elements 116, e.g. with the microarray, the comb-like structure 132 may be dipped into the wells 124 and/or into the vials 126 of the multi-well plate 128, e.g. the MTP. The wells 124 and/or the vials 126 and/or the multi-well plate 128, e.g. the MTP, may comprise and/or may be filled with the liquid test sample 114 and/or the at least one reagent 170, e.g. at least one assay reagent. The reagent 170 and/or the assay reagent may be an analyte-specific antibody, which may be digoxigenin-marked.

The method preferably may comprise one or more incubation steps, such as an incubation step having an incubation time. The incubation time may be a time period during which the test element 116 may stay in contact with the liquid test sample 114. The method, preferably the steps, may comprise at least one washing step 176. During the washing step 176, the test elements 116 may be washed, e.g. with at least one liquid and/or at least one washing buffer. During the washing step 176, unbound analytes and/or unbound antibodies, e.g. digoxigenin antibodies, may be removed from the test element 116.

After at least one suitable incubation time, the test elements 116, e.g. at least one microarray, may be washed. The washing step 176 may comprise removing the test element 116 out of the well 124 and/or the vial 126 and/or the multi-well plate 128, e.g. filled with the liquid test sample 114 with the analyte 112 and/or with the antibodies and/or at least one dipping into at least one well 124 and/or into at least one vial 126 and/or into at least one multi-well plate 128, preferably into at least one MTP well, filled with at least one washing buffer.

The method may comprise a first incubation time with the liquid test sample 114, preferably with the analyte 112 and the digoxigenin-labeled antibody 170 which specifically binds to the analyte 112, preferably followed by a first washing step 176, preferably followed by at least one second incubation time with anti-digoxigenin detection antibodies coupled to latex-particles (178, 180), preferably followed by at least one second washing step 176, preferably also in the MTP, preferably followed by at least one measurement of at least one signal, e.g. at least one fluorescence signal, preferably on the test element 116, e.g. on the solid carrier, for quantification of the respective analyte 112, preferably for determining binding of the said at least one analyte 112 to the analyzing zone 118, preferably on each test element 116, and/or for determining the change of the property of at least one part of the device 110 and/or of the liquid test sample 114. The detection antibodies may comprise fluorescent-marked anti-digoxigenin antibodies. During the incubation step and/or the washing step 176, preferably during all the incubation steps and/or during all the washing steps 176, the test elements 116, preferably the comb-like spaced structure 132, may be held in the MTP and/or in the multi-well plate 128 and/or in the well 124 and/or in the vial 126 and/or in the wells 124 and/or in the vials 126 by the test element holder 134, e.g. at least one suitable holder. The element holder preferably may hold and/or fix the test elements 116 perpendicular to shaking movements of the shaking unit 120, e.g. of at least one xy-shaker. The device 110, preferably the whole system, may comprise the sample holder 130, e.g. the MTP and/or the well 124 and/or the vial 126 and/or the multi-well plate 128, and/or the test element 116, e.g. the comb-like spaced structure 132, preferably a comb, and/or the test element holder 134, e.g. a holder. The system, preferably the liquid test sample 114 and/or the test element 116 and/or the antibodies and/or the analyte 112 and/or the reagent 170 and/or the washing buffer, may be mixed by the shaker unit, preferably by the xy-shaker.

The first incubation with the liquid test sample 114 may also comprise only the analyte 112, followed by a first washing step, and then followed by the incubation with the digoxigenin-labeled antibody 170, followed by a washing step, followed by the incubation with the anti-digoxigenin detection antibody coupled to latex particles (178, 180), followed by a washing step, followed by the measurement, e.g. by using the detector 156 to carry out the detection of the optical signal.

The reagent 170, e.g. the assay reagent, in the wells 124 and/or in the vials 126 and/or in the MTP and/or in the multi-well plate 128 and/or in the sample holder 130 may be added when the liquid test sample 114, preferably with the analyte 112, may be filled into the wells 124 and/or into the vials 126 and/or into the MTP and/or into the multi-well plate 128 and/or into the sample holder 130.

Alternatively or additionally, the reagent 170, e.g. the assay reagent, may be stored as liquid reagent, e.g. in at least one MTP, e.g. capped, preferably by at least one heat sealing film, which may be perforated when the liquid test sample 114, preferably with the analyte 112, may be filled into the wells 124 and/or into the vials 126 and/or into the MTP and/or into the multi-well plate 128 and/or into the sample holder 130.

Alternatively or additionally, the reagent 170, e.g. the assay reagent, may be stored, e.g. as dried and/or chemical reagent, wherein the reagent 170 may be solved during the use and/or the method, preferably by the liquid test sample 114 itself and/or by the shaking unit 120 and/or by at least one additional liquid.

The embodiment of the device 110 and/or the use and/or the method according to the present invention showed in figure 3A and/or figure 3B and/or figure 3C may be used for detecting Cardiac Troponin 140, e.g. Troponin T 140, as analyte 112. The device 110 may comprise at least one Troponin T 140 assay. Preferably more than one liquid test sample 114 may be tested for the analyte 112, preferably Troponin T 140, preferably for a concentration of Troponin T 140. In the device 110 showed in figure 3A, up to twelve, preferably twelve, liquid test samples 114, preferably with the analyte 112 Troponin T 140, may be analyzed. The liquid test samples 114 may be labeled by numbers, e.g. 1 to 12, and/or may be arranged in one row of the multi-well plate 128, preferably of the MTP. In the wells 124 and/or vials 126 of at least one first row 162 of the multi-well plate 128, preferably of the MTP, the liquid test samples 114 to be analyzed, e.g. the sample, and optionally at least one assay reagent, e.g. at least one antibody, e.g. anti-Troponin T antibodies, preferably labeled with digoxigenin, most preferably digoxigenin-labeled anti-Troponin T antibodies (Ab) ("Dig-MAK") 170 as listed in table 1 as MAK<TnT>M- 11-7-F(ab')2-Dig-(UE)5-DSS, may be arranged. Preferably, fluorescent labels and/or fluorescent markers may be introduced indirectly, e.g. in a following step, e.g. by incubation with a fluorescent anti-digoxigenin antibody, e.g. the third row. The wells 124 and/or the vials 126 of at least one second row 164 of the multi-well plate 128, preferably of the MTP, and/or of at least one fourth row 168 of the multi-well plate 128, preferably of the MTP, may be filled with washing buffer. At least one third row 166 of the multi-well plate 128, preferably of the MTP, may comprise and/or may be filled with at least one detection antibody, preferably with at least one fluorescent detection antibody, preferably a fluorescent anti-digoxigenin antibody. Different configurations of the MTP and/or of the multi-well plate 128, e.g. as Troponin T assay, may be possible. The first row 162 and/or the second row 164 and/or the third row 166 may be arranged according to the used numbering but may also be arranged in different order and/or embodiments with more or less rows and/or more or less columns may be possible. Figure 3A additionally shows schematically as boxes containing symbols the liquid test sample 114 labeled with number "1" and the liquid test sample 114 labeled with number "12" belonging to the respective wells 124 as indicated by the arrows. The liquid test sample 114 labeled with number "1" and the liquid test sample 114 labeled with number "12" exemplary both comprise at least one analyte 112, e.g. Troponin T 140, sketched by a diamond and at least one antibody, specifically at least one digoxigenin-labeled antibody as a reagent 170, sketched by a Y-shaped symbol with a label attached to the Y-shaped symbol.

Figure 3B shows an embodiment of an analyzing zone 118, e.g. combined by the third embodiment of the device 110 and/or by another embodiment of the device 110. The analyzing zone 118 may combine at least one microarray assembly. The analyzing zone 118 may comprise at least one surface 122, preferably at least one compact surface, preferably with 3 spots, e.g. spots with immobilized antibodies 138, most preferably comprising the at least one capture zone 136.

The capture zone 136, e.g. the spots, may have an average diameter 144 in the range of 100 µm to 800 µm, preferably of 700 µm. The analyzing zone 118 may comprise polystyrene, preferably black polystyrene. The analyzing zone 118 may have an average diameter 142 in the range from about 2 mm to about 8 mm, preferably of 4 mm. The analyzing zone 118 may have an average thickness 172 of 0.1 mm to 5 mm, preferably of 0.5 mm to 1 mm, most preferably of 0.8 mm.

Figure 3C shows a scheme for the use and the method of the third embodiment of the device 110, preferably figure 3C shows an assay principle, most preferably an assay principle for the Troponin T assay. It shows in particular four steps which may be comprised by the method and/or the use of the present invention connected by arrows. Figure 3C preferably shows a capture antibody, e.g. as immobilized antibody 138, e.g. the immobilized anti-Troponin T antibody (Ab), capture Ab ("Bi-MAK") as listed in table 1, and/or the generation of a sandwich, e.g. a connection of the immobilized antibody 138 with the analyte 112 and optionally the reagent 170, e.g. digoxigenin-labeled anti-Troponin T antibody ("Dig-MAK") as listed in table 1, a washing step 176, followed by an indirect fluorescence affinity labeling via the addition of anti-digoxigenin antibody which is fluorescently labeled with fluorescent latex particles 178, 180, e.g. as listed in table 1 as JG9-latex, again a washing step 176 and a readout, preferably by the detector 156.

Preferably, after adding and/or mixing, preferably twelve, liquid test samples 114, e.g. to and/or with the digoxigenin-labeled antibodies 170, e.g. the anti-Troponin antibodies, most preferably the digoxigenin-labeled anti-Troponin T antibodies (Ab) ("Dig-MAK") as listed in table 1, into the wells 124, preferably of the MTP, preferably of the first row 162, the comb-like spaced structure 132, preferably with twelve microarrays, may be dipped into the wells 124 and/or the vials 126 and/or may be incubated, e.g. in the incubation step, preferably at room temperature, e.g. regulated by the temperature control unit 152. Optionally, the incubation step also may comprise a control of the temperature comprising heating and/or cooling, preferably of the liquid test sample 114. In the case analyte 112 is present, the immobilized antibody 138 and the digoxigenin-labeled antibody may generate on the analyzing zone 118, preferably on the microarray, at least one complex, e.g. at least one sandwich complex, preferably at least one sandwich immunocomplex. After that, the test element 116, preferably the comb-like spaced structure 132, may be removed from and/or lifted out of the wells 124 and/or the vials 126 and may be dipped into the second row 164 of the MTP, preferably for washing away unbound components of the liquid test sample 114 and/or of the reagent 170 and/or of the test element 116. The test element 116, preferably the comb-like spaced structure 132, after this step may be dipped into the third row 166 of the MTP and may be incubated, preferably at room temperature. The sandwich complexes may be, e.g. fluorescently, marked with the aid of the digoxigenin group present in the reagent 170, e.g. via the digoxigenin group present in the sandwich antibody, preferably by the indirect labeling with fluorescent particles 180, preferably latex particles, e.g. fluorescent latex particles 178, which preferably may carry anti-digoxigenin antibodies on their surfaces, most preferably anti-digoxigenin latex particles ("JG9-latex") as listed in table 1. Preferably, after an additional washing step 176, preferably in the fourth row 168 of the MTP, the signal, e.g. a fluorescence of the microarrays, may be measured and/or detected, e.g. in a suitable setup, e.g. an optical setup comprising e.g. the at least one detector 156. The washing step may be performed using washing buffer, e.g. the washing buffer as listed in table 1. Preferably in a following step the concentration of the analyte 112 may be determined, e.g. using the signal and/or the evaluation unit 158 and/or the analyzing unit 154.

The device 110 and/or the system, e.g. comprising the comb and/or the comb holder and/or the MTP, may be shaked by the shaking unit 120, preferably by the xy-shaker, preferably at least during the whole incubation step, e.g. the incubation process, and/or the washing step 176, e.g. the washing process, preferably intensively shaked. The device 110 further may comprise at least one splash guard 174. In this embodiment, however, cross-contamination, e.g. contamination between different wells 124 and/or vials 126, seems to be not a problem, but the splash guard 174 may be useful in other embodiments of the present invention.

Preferably, the device 110, e.g. as assay device, and/or the method and/or the use, e.g. as assay process, comprising e.g. at the at least one incubation step and/or the at least one washing step 176 and/or the detection and/or a at least one output, e.g. of the concentration of the analyte 112, may be at least partially automatic, preferably completely automatic. The term "automatic" may refer to steps being processed without the help of a user and/or of an operator. Preferably, the user and/or the operator only may provide the liquid test sample 114. The device 110 and/or the method and/or the use may also at least partially be processed manually, e.g. by the user and/or the operator. The device 110 and/or the method and/or the use may also at least partially be processed by at least one platform, preferably by a platform processing the steps and the detection of a result automatically and/or integrated. The washing process, e.g. the washing steps 176, may be integrated.

In a preferred embodiment of the method and/or the use of the device 110 according to the present invention a first step may comprise a dipping of the test elements 116 in up to twelve wells 124 containing e.g. the liquid test samples 114, preferably with the reagents 170. In a second step the test elements 116 may be dipped into washing buffer. Preferably up to eight different steps may be possibly, preferably by using a multi-well plate 128 having eight rows and twelve columns of wells. The liquid test sample 114 may be at least one blood and/or serum and/or plasma sample.

Table 1 shows exemplary antibodies, reagents 170 and washing buffer as may be used in the device 110, the use and the method according to the present invention, preferably in the Troponin T assay, as described above.

**Table 1: Exemplary antibodies, reagents and washing buffer.**

| | |
|---|---|
| Digoxigenin-labeled anti-Troponin T Ab ("Dig-MAK") | MAK<TnT>M-11-7-F(abʹ)2-Dig-(UE)5-DSS: |
| | Ab fragment of mouse IgG, labelled with digoxigenin-(UE)5-(DSS) at lysines (molar Ab:label-ratio used for conjugation 1:5). |
| | Storage buffer: |
| | PBS pH 8.0 / 6% Saccharose |
| | Dilution/working buffer: |
| | 50mM HEPES pH 7.4 / 1% RPLA new / 0,15% Synperonic P85 / 4% Saccharose / 200mM NaCl / 3mg/ml MAK<CK-MM>M33-IgG, 2 mg/ml MAK<CK-MM>M33-IgG1/Fab'1-Poly |
| | Working concentration: 300µg/ml |
| Immobilized anti-Troponin T Ab, capture Ab ("Bi-MAK") | MAK<TnT>Chim-5D8-F(abʹ)₂-Bi-((UE)5-DSS)-Cy3: |
| | Chimeric Ab fragment of humanized mouse IgG, labelled with digoxigenin-(UE)5-DSS at lysines (molar Ab:label-ratio used for conjugation 1:5) and additionally with Cy3 |
| | (Osu) for in-process-control purposes |
| | Storage buffer: |
| | KP 50mM pH 7.5 / NaCl 150mM / 6.5% Saccharose |
| | Working/spotting buffer: |
| | 5mM MES, 6mM Tris, 1% Saccharose, 0.2% RPLA, 0.01% Tween, pH 7.65, 0.0095% NaN3 |
| | Working concentration: 250µg/ml |
| Anti-digoxigenin latex particles ("JG9-latex") | CM-Lx(JG9)-MAK<Dig>M-19-11-IgG-poly: |
| | Carboxymethyl latex particles (200nm diameter), dyed with JG9 (5-6 µmol/g latex), conjugated with the polymeric form of anti-digoxigenin Ab MAK<Dig>M-19-11-IgG (200mg Ab/g latex) via sNHS/EDC and glycin stop |
| | Storage buffer: |
| | 5mM KP pH 7,4, 1% RPLA new, 0.01% MIT, 0.25% CAA |
| | Dilution/working buffer: |
| | TAPS 62.5mM pH 8.5 / NaCl 1.25 M / Tween 20 0.06% / RPLA4 0.3 5% / NaN3 0.0009 % / 12.5µg/ml MAK<CK-MM>M33-IgG, 12.5µg/ml MAK<CK-MM>M33-IgG1/Fab'1-Poly |
| | Working concentration: 0.1% |
| Washing buffer | 10mM Tris/HCl pH 8.0, 0.1% Triton X-100, 0.01% Oxipyrion, 0.001% MIT |

### List of reference numbers

- 110: device
- 112: analyte
- 114: liquid test sample
- 116: test element
- 118: analyzing zone
- 120: shaking unit
- 122: surface
- 124: well
- 126: vial
- 128: multi-well plate
- 130: sample holder
- 132: comb-like structure
- 134: test element holder
- 136: capture zone
- 138: immobilized antibody
- 140: cardiac Troponin
- 142: average diameter analyzing zone
- 144: average diameter capture zone
- 146: drive unit
- 148: z-stage
- 150: xy-stage
- 152: temperature control unit
- 154: analyzing unit
- 156: detector
- 158: evaluation unit
- 160: data processor
- 162: first row
- 164: second row
- 166: third row
- 168: fourth row
- 170: reagent
- 172: average thickness
- 174: splash guard
- 176: washing step
- 178: fluorescent latex particle
- 180: fluorescent particle
- 182: x-direction
- 184: y-direction
- 186: z-direction
- 188: coordinate system

## Claims

1. A device (110) for detecting at least one analyte (112) in at least one liquid test sample (114) using a plurality of test elements (116), wherein the device (110) comprises the plurality of test elements (116), wherein each of the test elements (116) individually can be brought into contact with the liquid test sample (114), wherein each of the plurality of test elements (116) comprises at least one analyzing zone (118) for detecting the at least one analyte (112), wherein the device (110) further comprises a shaking unit (120), the shaking unit (120) being capable of generating a relative flow of the liquid test sample (114), the relative flow having at least one flow component perpendicular to a surface (122) of the analyzing zone (118).

2. The device (110) according to the preceding claim, wherein the analyzing zone (118) is non-porous.

3. The device (110) according to one of the preceding claims, wherein the device (110) further comprises at least one well (124) for receiving the liquid test sample (114), wherein the well (124) comprises at least one vial (126).

4. The device (110) according to the preceding claim, wherein the device (110) further comprises at least one sample holder (130) for holding the well (124) for receiving the liquid test sample (114).

5. The device (110) according to the two preceding claims, wherein the sample holder (130) comprises the multi-well plate (128).

6. The device (110) according to the two preceding claims, wherein the shaking unit (120) is adapted to shake the sample holder (130).

7. The device (110) according to one of the preceding claims, wherein the plurality of test elements (116) form a comb-like spaced structure (132).

8. The device (110) according to one of the preceding claims, wherein the device (110) comprises at least one test element holder (134), wherein the test elements (116) are connected to the test element holder (134).

9. The device (110) according to one of the preceding claims, wherein each test element (116) comprises a plurality of analyzing zones (118).

10. The device (110) according to one of the preceding claims, wherein the analyzing zone (118) comprises at least one capture zone (136).

11. The device (110) according to the preceding claim, wherein the capture zone (136) comprises at least one immobilized antibody (138).

12. The device (110) according to the preceding claim, wherein the immobilized antibody (138) is an antibody which specifically recognizes a cardiac Troponin (140).

13. The device (110) according to the four preceding claims, wherein the analyzing zone (118) is spot-like and has an average diameter (142) in the range from about 2 mm to about 8 mm and the at least one capture zone (136) comprised by the analyzing zone (118) is spot-like and has an average diameter (144) in the range from about 100 µm to about 800 µm.

14. The device (110) according to one of the preceding claims, wherein the device (110) comprises at least one drive unit (146) which is capable of bringing into contact the plurality of test elements (116) with a plurality of liquid samples (114), wherein the plurality of liquid samples comprises the at least one liquid test sample.

15. Use of a device (110) according to one of the preceding claims for detecting at least one analyte (112) in at least one liquid test sample (114).

16. A method for detecting at least one analyte (112) in at least one liquid test sample (114), the method using the device (110) according to one of the preceding claims referring to a device (110) for detecting at least one analyte (112) in at least one liquid test sample (114).

17. The method according to the preceding claim, wherein the method comprises the steps of:
a) providing at least one liquid test sample (114) and a plurality of test elements (116);
b) contacting each of the test elements (116) individually with the liquid test sample (114), wherein each of the plurality of test elements (116) comprises at least one analyzing zone, wherein the analyzing zone (118) detects the at least one analyte (112), preferably under conditions which allow for binding and/or at least one change of at least one property of at least one part of the device (110) and/or of the liquid test sample (114), wherein the step comprises generating a relative flow of the liquid test sample (114) by at least one shaking unit (120), the relative flow having at least one flow component perpendicular to a surface (122) of the analyzing zone (118); and
c) determining binding of the said at least one analyte (112) to the analyzing zone (118) on each test element (116) and/or the change of the property of at least one part of the device (110) and/or of the liquid test sample (114).

18. The method according to one of the preceding method claims, wherein the liquid test sample (114) is at least one blood and/or serum and/or plasma sample.
